Europäisches Patentamt

European Patent Office

Office européen des brevets

(19)

(11) Numéro de publication: **0 430 736 A1**

## (12) DEMANDE DE BREVET EUROPEEN

(21) Numéro de dépôt: 90403131.7

(22) Date de dépôt: 06.11.90

(51) Int. Cl.⁵: **A23L 1/30**, A61K 35/72, A61K 35/74

(30) Priorité: 20.11.89 FR 8915197

(43) Date de publication de la demande:
05.06.91 Bulletin 91/23

(84) Etats contractants désignés:
**BE CH ES IT LI LU NL SE Bulletin**

(71) Demandeur: **LABORATOIRES DOLISOS**
**71 rue Beaubourg**
**F-75003 Paris(FR)**

(72) Inventeur: **Levy, françois**
**38 rue Boileau**
**F-75016 Paris(FR)**

(74) Mandataire: **Madeuf, Claude Alexandre Jean**
**et al**
**CABINET MADEUF 3, avenue Bugeaud**
**F-75116 Paris(FR)**

(54) **Produit à base de levures diététiques permettant d'obtenir un meilleur équilibre des fonctions organiques humaines et animales.**

(57) Du Bifido Bactérium Bifidum (Bifidus) est à ajouter à une quantité déterminée de levure lactique et/ou de levure de bière.

EP 0 430 736 A1

## PRODUIT À BASE DE LEVURES DIÉTÉTIQUES PERMETTANT D'OBTENIR UN MEILLEUR ÉQUILIBRE DES FONCTIONS ORGANIQUES HUMAINES ET ANIMALES.

La présente invention concerne un produit à base de levures diététiques permettant d'améliorer l'équilibre des fonctions organiques tant chez l'homme que chez les animaux.

On sait que la levure lactique et la levure de bière sont des levures diététiques qui sont riches en protéines, en vitamines du groupe B et en oligo-éléments. Ces levures favorisent le métabolisme des glucides et des lipides. Elles sont connues pour présenter une action bénéfique sur l'état de la peau, des ongles et des cheveux ainsi que sur la régulation du transit intestinal. Chez les animaux, ces levures améliorent la qualité du poil.

Par ailleurs, le Bifido Bacterium Bifidum, appelé également Bifidus dans ce qui suit, présente une action rééquilibrante sur la flore intestinale en freinant la multiplication des micro-organismes indésirables dans l'intestin. En outre, le Bifidus améliore l'éclat du teint et la qualité de la peau.

Il a maintenant été constaté d'une manière surprenante qu'une association particulière de telles levures diététiques avec du Bifidus présente une action synergique et renforce l'équilibre des fonctions organiques tant chez l'homme que chez les animaux.

Mais, il y a lieu de considérer que l'on connaissait antérieurement à cette invention des publications montrant, soit des produits diététiques, soit des médicaments à base également de levures diététiques ou de levures de bière, c'est pourquoi il est particulièrement fait référence au FR-M-3614 qui a pour objet une composition anhydre contenant un produit à base de lait, un extrait de malte placé dans une masse de sucre substantiellement anhydre qui contient en même temps des cultures déshydratées de germes divers, le tout pouvant être présenté sous les formes habituelles en pharmacie pour l'absorption.

Conformément à l'invention, le produit à base de levures diététiques se présentant sous la forme de comprimés à croquer, de pastilles à avaler, de poudre ou de paillettes à consommer éventuellement avec les aliments est caractérisé en ce qu'il comprend une proportion de levures allant d'environ 100 % de levure lactique pour 0 % de levure de bière à 0 % de levure lactique pour environ 100 % de levure de bière, auxquelles on incorpore de 0,002 % à 15 % de Bifido Bacterium Bifidum (Bifidus).

On préfère toutefois employer une proportion de levures allant d'environ 50 % de levure lactique pour 50 % de levure de bière, à environ 1/3 de levure lactique pour 2/3 de levure de bière, auxquelles on incorpore de 0,02 à 5 % de Bifido Bacterium Bifidum (Bifidus).

Diverses autres caractéristiques de l'invention ressortent d'ailleurs de la description détaillée qui suit.

Des formes de réalisation de l'objet de l'invention sont indiquées ci-après à titre d'exemples non limitatifs.

On a préparé des comprimés à croquer de préférence au cours des repas et répondant à la formule suivante :
- levure lactique 50 %
- levure de bière 49,998 %
- Bifidus 0,002 %
- Excipient QSP : un comprimé

On a préparé des pastilles à avaler de préférence avant les repas et répondant à la formule suivante :
- levure lactique 34,98 %
- levure de bière 65 %
- Bifidus 0,02 %
- Excipient QSP : une pastille

On a préparé une poudre à consommer de préférence comme assaisonnement avec des crudités et répondant à la formule suivante :
- levure lactique 44 %
- levure de bière 51 %
- Bifidus 5 %
- Excipient QSP : poudre

On a préparé des paillettes à mélanger de préférence dans du yogourt consommé à la fin d'un repas et répondant à la formule suivante :
- levure lactique 33 %
- levure de bière 57 %
- Bifidus 10 %
- Excipient QSP : paillettes

On a préparé des pastilles à sucer de préférence à jeun et répondant à la formule suivante :
- levure lactique 85 %
- Bifidus 15 %
- Excipient QSP : pastilles

On a préparé une poudre à mélanger à de l'eau et répondant à la formule suivante :
- levure de bière 99,8 %
- Bifidus 0,2 %
- Excipient QSP : poudre

En outre et bien que les exemples ci-dessus concernent la médecine humaine, le produit conforme à l'invention peut aussi bien être utilisé en médecine animale dans des proportions analogues.

Après quelques jours de traitement à l'aide des diverses préparations ci-dessus, on a constaté de manière surprenante un meilleur équilibre des fonctions organiques des personnes ou des animaux ayant subi le traitement qui a pu être poursuivi plusieurs mois.

Cette amélioration est en effet bien supérieure à ce que l'on pouvait attendre d'une simple juxtaposition des propriétés indiquées précédemment des levures diététiques (levure lactique et/ou levure de bière) et du Bifido Bacterium Bifidum (Bifidus) du fait de la synergie existant entre ces produits.

## Revendications

1 - Produit à base de levures diététiques se présentant sous la forme de comprimés à croquer, de pastilles à avaler, de poudre ou de paillettes à consommer éventuellement avec les aliments, caractérisé en ce qu'il comprend une proportion de levures allant d'environ 100 % de levure lactique pour 0 % de levure de bière à 0 % de levure lactique pour environ 100 % de levure de bière, auxquelles on incorpore de 0,002 % à 15 % de Bifido Bacterium Bifidum (Bifidus).

2 - Produit à base de levures diététiques selon la revendication 1, caractérisé en ce qu'il comprend une proportion de levures allant d'environ 50 % de levure lactique pour 50 % de levure de bière à environ 1/3 de levure lactique pour 2/3 de levure de bière, auxquelles on incorpore de 0,02 à 5 % de Bifido Bacterium Bifidum (Bifidus).

3 - Produit à base de levures diététiques selon l'une des revendications 1 et 2, caractérisé en ce qu'il présente la formulation suivante en produit actif :
- levure lactique 50 %
- levure de bière 49,998 %
- Bifidus 0,002 %

4 - Produit à base de levures diététiques selon l'une des revendications 1 et 2, caractérisé en ce qu'il présente la formulation suivante en produit actif :
- levure lactique 34,98 %
- levure de bière 65 %
- Bifidus 0,02 %

5 - Produit à base de levures diététiques selon l'une des revendications 1 et 2, caractérisé en ce qu'il présente la formulation suivante en produit actif :
- levure lactique 44 %
- levure de bière 51 %
- Bifidus 5 %

6 - Produit à base de levures diététiques selon l'une des revendications 1 et 2, caractérisé en ce qu'il présente la formulation suivante en produit actif :
- levure lactique 33 %
- levure de bière 57 %
- Bifidus 10 %

7 - Produit à base de levures diététiques selon l'une des revendications 1 ou 2, caractérisé en ce qu'il présente la formulation suivante en produit actif :
- levure lactique 85 %
- Bifidus 15 %
- Excipient QSP : pastilles

8 - Produit à base de levures diététiques selon l'une des revendications 1 ou 2, caractérisé en ce qu'il présente la formulation suivante en produit actif :
- levure de bière 99,8 %
- Bifidus 0,2 %
- Excipient QSP : poudre

**Office européen
des brevets**

**RAPPORT DE RECHERCHE
EUROPEENNE**

Numéro de la demande

**EP 90 40 3131**

# DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-M-3 614 (SIGURTA)<br>* page 2, colonne 2, alinéa 4 - page 3, colonne 2, alinéa 3 *<br>– – – | 1 | A 23<br>L 1/30<br>A 61 K 35/72<br>A 61 K 35/74 |
| A | FR-M-6 779 (BLAISE)<br>* page 2, colonne 1, alinéa 4 - page 2, colonne 2, alinéa 7 *<br>– – – | 1 | |
| A,E,A | WO-A-8 900 425 (ADVANCE)<br>* revendications 1, 2, 3, 4, 5, 6 & EP-A-396744 *<br>– – – | 1 | |
| A | US-A-4 251 519 (ROBBINS ET AL)<br>* revendication 1 *<br>– – – | 1 | |
| A | EP-A-0 181 170 (ADVANCE)<br>* revendication 1 *<br>– – – | 1 | |
| A | WO-A-8 901 025 (ALLNATUR BIO-PRODUKTE)<br>* revendications 1, 9 *<br>– – – | 1 | |
| A | FR-M-2 946 (SALVOXYL)<br>* le document en entier *<br>– – – – – | 1 | |

**DOMAINES TECHNIQUES RECHERCHES (Int. Cl.5)**

A 23 L
A 61
K
A 21 K

Le présent rapport de recherche a été établi pour toutes les revendications

| Lieu de la recherche | Date d'achèvement de la recherche | Examinateur |
|---|---|---|
| La Haye | 07 mars 91 | VAN MOER A.M.J. |